(19) Europäisches Patentamt
European Patent Office
Office européen des brevets

(11) Veröffentlichungsnummer: **0 263 437**
**B1**

(12) # EUROPÄISCHE PATENTSCHRIFT

(45) Veröffentlichungstag der Patentschrift: **30.01.91**

(51) Int. Cl.$^5$: **C 07 D 405/12,**
C 07 D 409/12, C 09 K 19/58,
G 02 F 1/13

(21) Anmeldenummer: **87114332.7**

(22) Anmeldetag: **01.10.87**

(54) **Chirale Aryl-2,3-epoxyalkyl-ether und deren entsprechende Thioverbindungen und ihre Verwendung als Dotierstoff in Flüssigkristall-Phasen.**

(30) Priorität: **06.10.86 DE 3633968**

(43) Veröffentlichungstag der Anmeldung:
**13.04.88 Patentblatt 88/15**

(45) Bekanntmachung des Hinweises auf die Patenterteilung:
**30.01.91 Patentblatt 91/05**

(84) Benannte Vertragsstaaten:
**AT BE CH DE ES FR GB IT LI NL SE**

(56) Entgegenhaltungen:
**WO-A-87/02036**

**D.W. Walba, 11th International Liquid Crystal Conference, Scientific Sessions and Abstracts Clark Kerr Campus University of California at Berkeley 30.06.-04.07.86**

(73) Patentinhaber: **HOECHST AKTIENGESELLSCHAFT**
**Postfach 80 03 20**
**D-6230 Frankfurt am Main 80 (DE)**

(72) Erfinder: **Hemmerling, Wolfgang, Dr.**
**Billtalstrasse 32**
**D-6231 Sulzbach (DE)**
Erfinder: **Dübal, Hans-Rolf, Dr.**
**Kirschgartenstrasse 10**
**D-6238 Hofheim am Taunus (DE)**
Erfinder: **Müller, Ingrid, Dr.**
**Am Pfingstbrunnen 1**
**D-6238 Hofheim am Taunus (DE)**
Erfinder: **Ohlendorf, Dieter, Dr.**
**Am Kühlen Grund 4**
**D-6237 Liederbach (DE)**
Erfinder: **Wingen, Rainer, Dr.**
**Rotkäppchenweg 10**
**D-6234 Hattersheim am Main (DE)**

**EP 0 263 437 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Flüssigkristalle haben insbesondere im letzten Jahrzehnt Eingang in verschiedenste technische Gebiete gefunden, in denen elektrooptische und Anzeigevorrichtungs-Eigenschaften gefragt sind (z.B. in Uhren-, Taschenrechner- und Schreibmaschinenanzeigen). Diese Anzeigevorrichtungen beruhen auf den dielektrischen Ausrichtungseffekten in den nematischen, cholesterischen und/oder smektischen Phasen der flüssigkristallinen Verbindungen, wobei — verursacht durch die dielektrische Anisotropie — die molekulare Längsachse der Verbindungen eine bevorzugte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen, die an sich für die Technik wegen ihrer einzigartigen Eigenschaften sehr vielversprechende chemische Verbindungen sind, eher zu langsam. Dieser Nachteil macht sich insbesondere dann bemerkbar, wenn — was bei größeren Anzeigeelementflächen zwangsläufig der Fall ist — eine große Anzahl von Bildpunkten angesteuert werden muß, wodurch die Produktionskosten solcher, diese größeren Flächen enthaltenden Geräte wie Videogeräte, Oszillographen oder TV-, Radar-, EDV- oder Schreibautomaten-Bildschirme zu hoch wären.

Neben den nematischen und cholesterischen Flüssigkristallen haben seit einigen wenigen Jahren in zunehmendem Maße auch für Praxisanwendungen geneigt("tilted")-smektische Flüssigkristall-Phasen an Bedeutung gewonnen. Wenn solchen geneigt-smektischen Phasen, insbesondere smektisch C ($S_c$ oder SmC)Phasen, geeignete Dotierstoffe zugesetzt werden, die eine sogenannte spontane Polarisation ($P_s$) zeigen oder in der Flüssigkristall-Phase induzieren, so können die Phasen in eine ferroelektrische Flüssigkristall-Phase umgewandelt werden (Benennung von $P_s$ in $nC\cdot cm^{-2}$), siehe dazu beispielsweise Lagerwall et al. im Aufsatz (Ferroelectric Liquid Cristals for Displays" (Ferroelektrische Flüssigkristalle für Anzeigeelemente), SID Symposium, October Meeting, 1985, San Diego (USA). Diese ferroelektrischen Flüssigkristall-Phasen weisen — vergleichen mit den üblichen TN("twisted nematic")-Zellen — um etwa den Faktor 1000 schnellere Schaltzeiten auf, so daß sie — auch aufgrund anderer positiver Eigenschaften wie einer bistabilen Schaltmöglichkeit — gute potentielle Kandidaten für die oben aufgeführten Anwendungsgebiete (z.B. über eine Matrixansteuerung) sind.

Auf der 11. Internationalen, Flüssigkristallkonferenz (30.6. bis 4.7.1986) in Berkeley/USA wurden von D. W. Walba ferroelektrische Flüssigkristalle vorgestellt, die chirale 2,3-Epoxy-alkyl-Seitenketten enthalten und folgende allgemeine Formel aufweisen:

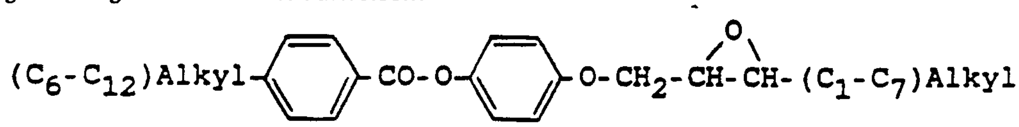

Die Verbindung mit einem $C_{10}$-Alkyl- bzw. $C_3$-Alkylrest weist zwischen 75°C und 80°C eine SmC*-Phase auf; die Schaltzeit (75°C, 15 V/µm) beträgt 14 µsec, der Wert für die spontane Polarisation $P_s$ beträgt 45 $nC/cm^2$.

Aufgabe der vorliegenden Erfindung ist es, Verbindungen aufzuzeigen, die bei hohen Werten für die eigene oder in Flüssigkristall-Phasen induzierte spontane Polarisation $P_s$ Strukturelemente aufweisen, die sie auch mit anderen Komponenten in Flüssigkristall-Systemen "verträglich" (d. h. mischbar) machen, da u.a. de mesogene Teil der Moleküle oftmals für eine gute "Verträglichkeit" mit den anderen Mischungskomponenten in Flüssigkristall-Systemen verantwortlich ist; dabei müssen diese Verbindungen selbst nicht unbedingt flüssigkristallin sein, insbesondere nicht unbedingt eine SmC-Phase aufweisen.

Die Erfindung geht aus von den bekannten chiralen Verbindungen mit einem mesogenen aromatischen Baustein und einem chiralen Baustein mit einem heterocyclischen Dreiring. Die erfindungsgemäßen Verbindungen sind dann dadurch gekennzeichnet, daß in der allgemeinen Formel (I)

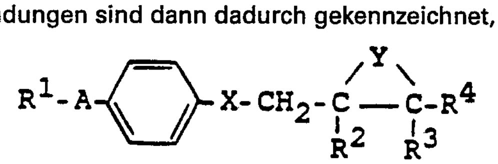
(I)

die Symbole folgende Bedeutung haben:

$R^1$ = geradkettiges oder verzweigtes $(C_1—C_{12})$Alkyl, wobei eine oder zweit nicht-benachbarte $CH_2$-Gruppen durch 0- und/oder S-Atome ersetzt sein können,

A = Diazin-2,5-diyl oder Diazin-3,6-diyl,

X, Y = O und/oder S, und

$R^2$, $R^3$, $R^4$ = unabhängig voneinander H, geradkettiges $(C_1—C_{10})$Alkyl oder verzweigtes $(C_3—C_{10})$Alkyl, wobei $R^2$, $R^3$ und $R^4$ nicht gleichzeitig H sind.

Die N-Atome im Diazin-Ringsystem können sich dabei in 1,3-(Pyrimidine) oder in 1,2-Stellung (Pyridazine) befinden, wobei in der bevorzugten 1,3-Stellung sich die N-Atome auf der dem Rest $R^1$ zugewandten Seite oder der dem Phenylring zugewandten Seite des Ringsystems befinden können.

Unter den Verbindungen der allgemeinen Formel (I) sind die bevorzugt, bei denen die Symbole folgende Bedeutung haben: $R^1$ = geradkettiges $(C_5—C_{11})$Alkyl, wobei eine $CH_2$-Gruppe durch ein O- oder S-Atom ersetzt sein kann, X, Y = O, $R^2$, $R^3$ = H und $R^4$ = geradkettiges oder verzweigtes $(C_3—C_7)$Alkyl.

Die Verbindungen der allgemeinen Formel (I) können beispielsweise hergestellt werden durch Umsetzung der Phenole oder Thiophenole der allgemeinen Formeln (II), (III) oder (IV) mit dem Oxiranen oder Thiiranen der allgemeinen Formel (V); worin Z = H ist oder für eine nucleofuge Gruppe steht:

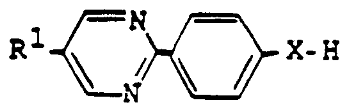

(II)

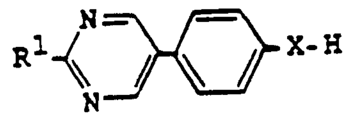

(III)

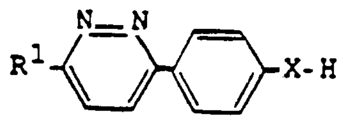

(IV)

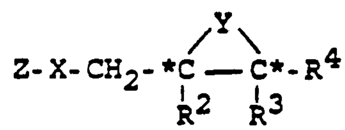

(V)

Die Verknüpfung der mesogenen Phenole oder Thiophenole der allgemeinen Formeln (II), (III) oder (IV) mit den chiralen Oxiranen oder Thiiranen der allgemeinen Formel (V) kann in an sich bekannter Weise erfolgen, z.B. durch Umsetzung von (II), (III) oder (IV) mit den Epoxyalkoholen (Y = O, X = O und Z = H) unter Zuhilfenahme von Diethylazodicarboxylat und Triphenylphosphin, wie von Mitsonobu, "The Use of Diethyl Azodicarboxylate and Triphenylphosphine in Synthesis and Transformation Natural Products" in Synthesis 1981, S.1—28 beschrieben. Möglich ist es auch, die Epoxyalkohole mit einer derartigen nucleofugen Gruppe Z zu versehen, daß die Umsetzung mit Alkali oder Erdalkali-Salzen der Verbindungen (II), (III) oder (IV) zur Bildung der gewünschten Ether der allgemeinen Formel I (mit X = O) führt. Als derartige nucleofuge (Abgangs-)Gruppen sind u.a. Tosylate, Brosylate, Mesilate oder Triflate dem Fachmann bekannt, sie lassen sich in bekannter Weise z.B. aus den Alkoholen und den jeweiligen Säurechloriden herstellen. Entsprechende Reaktionsmöglichkeiten gelten auch für die Thiiranverbindungen.

Die Edukte sind aus der Literatur bekannte Verbindungen. Beispielsweise können die Verbindungen (II) mit X = O durch Kondensation von substituierten Benzamiden mit 2-Alkylmalonsäureestern, Überführung der entstehenden Dihydroxypyrimidine in Dichlorpyrimidine und nachfolgende Hydrogenolyse hergestellt werden (siehe DE—C 22 57 588). Die Verbindungen (III) mit X = O werden beispielsweise durch Kondensation entsprechend substituierter 2-Aryl-3-(methylthio)acroleine mit geeigneten Amidinen hergestellt [siehe Kano et al., "A New and Facile Synthesis of 5-Arylpyrimidines and 4-Arylpyrazoles" in Heterocycles, Vo. 19, No. 6, 1079 bis 1082 (1982)]. Aus den Phenolen werden die entsprechenden Thiophenole nach bekannten Methoden erhalten [z.B. Newman et al., "The Conversion of Phenols to Thiphenoles via Dialkylcarbamates" in J. Org. Chem., *31*, S. 3980—3984 (1966)].

Die Oxirane (Epoxyalkohole) als bevorzugte Verbindungen den allgemeinen Formel (V) mit X, Y = O und Z = H werden beispielsweise aus den entsprechenden Allylalkoholen durch enantioselektive Epoxidierung hergestellt (siehe Pfenninger, "Asymmetric Epoxidation of Allylic Alcohols: The Skarplen Epoxidation" in Synthesis 1986, S. 89—116). Sie werden dann als solche eingesetzt (Z = H) oder aber nach Standardmethoden, z.B. durch Umsetzung mit 4-Toluolsulfonylchlorid, in die entsprechenden Tosylate überführt (Z = $SO_2C_6H_4CH_3$), analoges gilt für die anderen genannten nucleofugen Gruppen.

Eine weitere Lösung der gestellten Aufgabe ist eine verdrillbare Flüssigkristall-Phase mit einem Gehalt an mindestens einer chiralen Verbindung, die als chirale Verbindung mindestens eine Verbindung der allgemeinen Formel (I), enthält. Untrer dem Begriff "verdrillbare Flüssigkristall-Phase" sind nematische, cholesterische oder geneigt("tilted")-smektische, insbesondere SmC-Phasen zu verstehen.

Die verdrillbaren Flüssigkristall-Phasen bestehen aus 2 bis 20, vorzugsweise 2 bis 15 Komponenten, darunter mindestens einer der erfindungsgemäß beanspruchten chiralen Verbindungen. Die anderen Bestandteile werden vorzugsweise ausgewählt aus den bekannten Verbindungen mit nematischen cholesterischen und/oder geneigt-smektischen Phasen, dazu gehören beispielsweise Schiffsche Basen, Biphenyle, Terphenyle, Phenylcyclohexane, Cyclohexylbiphenyle, Pyrimidine, Zimtsäureester, Cholesterinester, verschieden überbrückte, terminal-polare mehrkernige Ester von p-Alkylbenzoesäuren. Im allgemeinen liegen die im Handel erhältlichen Flüssigkristall-Phasen bereits vor der Zugabe der chiralen Verbindung(en) als Gemische verschiedenster Komponenten vor, von denen mindestens eine mesogen ist, d.h. als Verbindung, in derivatisierter Form oder im Gemisch mit bestimmten Cokomponenten eine Flüssigkristall-Phase zeigt [=mindestens eine enantiotrope (Klärtemperatur $>$ Schmelztemperatur) oder monotrope (Klärtemperatur $<$ Schmelztemperatur) Mesophasenbildung erwarten läßt].

Insbesondere enthält die verdrillbare Flüssigkristall-Phase neben mindestens einer der erfindungsgemäß beanspruchten chiralen Verbindungen eine Phenylpyrimidinverbindung mit $S_c$-Phase, z.B. ein 4-(5-Alkyl-pyrimidin-2-yl)-1-alkoxybenzol.

Von der oder den erfindungsgemäßen Verbindung(en) enthalten die Flüssigkristall-Phasen im allgemeinen 0,01 bis 70 Gew.-%, insbesondere 0,05 bis 50 Gew.-%.

Die erfindungsgemäßen Verbindungen sind insbesondere als Dotierstoffe für geneigt-smektische Flüssigkristall-Phasen geeignet, da sie diese in ferroelektrische Flüssigkristall-Phasen umwandeln; die Werte für die spontane Polarisation (Ps) liegen im Bereich von etwa 30 bis 70 $nC \cdot cm^{-2}$ (extrapoliert auf die reine Verbindung).

BEISPIELE

Herstellungsbeispiele 1 bis 8

(2S, 3S)-(-)-2-[4-(5-Octyl-pyrimidin-2-yl)phenyloxy]methyl-3-propyl-oxiran (Beispiel 1)

Eine Lösung von 1,42 g (5 mmol) des 2-(4-Hydroxy-phenyl)-5-n-oxtyl-pyrimidins in 50 ml Dimethylformamid wird bei einer Temperatur von 0°C mit 0,16 g NaH (5 mmol), als 80 %ige Dispersion in Öl, versetzt. Nach Abklingen der Gasentwicklung werden 1,35 g (7 mmol) des (2S, 3S)-1-(Mesyloxy-2,3-epoxyhexans zugegeben und bei 0°C während 6 h gerührt. Anschließend wird in 100 ml Eiswasser hydrolysiert und mit Dichlormethan extrahiert. Nach dreimaliger Säulenchromatographie (Kieselgel, Dichlormethan) werden 0,61 g (32% d.Th.) an farblosen Kristallen erhalten. Das IR-Spektrum (in KBr) und das $^1H$-NMR-Spektrum (in $CDCl_3$) stützen die angegebene Strukturformel, der Drehwert beträgt $[\alpha]_D^{25}$: −18,8° (c = 10, $CH_2Cl_2$).

Bei Einsatz der entsprechenden Phenole (II), (III) oder (IV) und der Epoxyalkohole (V) sind diese und die nachstehende Vorschrift repräsentativ für alle Verbindungen der nachfolgenden Tabelle (I).

(2S, 3S)-(-)-2-[4-(5-Nonyl-pyrimidin-2-yl)phenyloxy]methyl-3-pentyl-oxiran (Beispiel 4)

Es werden 1,31 g (5 mmol) Triphenylphosphin und 0,79 g (5 mmol) Azodicarbonsäurediethylester in 10 ml Tetrahydrofuran bei 0°C vereinigt. Nach 20 min werden 1,49 g (5 mmol) des 2-(4-Hydroxy-phenyl)-5-n-nonyl-pyrimidins und 0,72 g (5 mmol) des (2S, 3s)-2,3-Epoxyoctanols jeweils in 8 ml Tetrahydrofuran, zugegeben. Nach 24 h wird im Vakuum zur Trockene eingedampft und der Rückstand wird über Kieselgel (200 g) mit $CH_2Cl_2$ chromatographiert. Nach Umkristallisation aus Hexan werden 0,79 g an farblosen Kristallen erhalten; $[\alpha]_D^{25}$: −15,3° (c = 5, $CHCl_3$).

Tabelle I

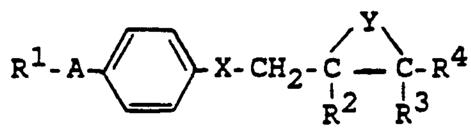

(I)

$(X,Y=O;\ R^2,R^3=H)$

| Bsp.-Nr. | A | $R^1$ | $R^4$ | Phasenfolge *) (°C) K | $S_B$ | $S_A$ | I |
|---|---|---|---|---|---|---|---|
| 1 | 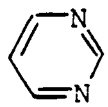 | $H_{17}C_8$ | $C_3H_7$ | · 90 | - | - | · |
| 2 | " | $H_{17}C_8$ | $C_4H_9$ | · 98 | - | - | · |
| 3 | " | $H_{17}C_8$ | $C_5H_{11}$ | · 106 | - | - | · |
| 4 | " | $H_{19}C_9$ | $C_5H_{11}$ | · 104 | - | - | · |
| 5 | " | $H_{21}C_{10}$ | $C_3H_7$ | · 88 | - | - | · |
| 6 | " | $H_{21}C_{10}$ | $C_7H_{15}$ | · 107 | - | - | · |
| 7 | 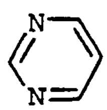 | $H_{17}C_8$ | $C_3H_7$ | · 55 | · 90 | · 102 | · |
| 8 | " | $H_{17}C_8$ | $C_5H_{11}$ | · 70 | · 101 | · 104 | · a) |

*) K = kristallin, S = smektisch, I = isotrop

a) statt $S_B$ muß in diesem Fall $S_F$ stehen

Anwendungsbeispiel 1 bis 8

Zur Überprüfung der Wirksamkeit der vorstehend beschriebenen Verbindungen als Dotierstoffe in Flüssigkristall-Systemen werden diese n Konzentrationen von jeweils 10 Mol-% (oder in einem Fall von 25 Mol-%) mit dem Racemat der Verbindung

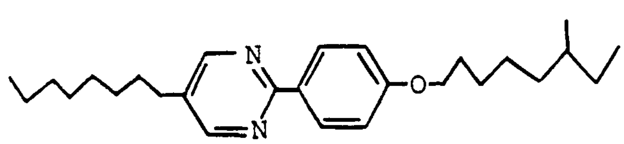

4-(5-Octyl-pyrimidin-2-yl)-1-(6-methyl-oct-1-oxy)benzol gemischt und jeweils die Werte für die spontane Polarisation ($P_s$ in $nC \cdot cm^{-2}$), für die Schaltzeit τ (in μs) und für den optischen Neigungswinkel der SmC*-Phase θ (in °) der Mischung bestimmt.

Die $P_s$-Werte werden nach der Methode von Sawyer et al. (Phys. Rev. *35*, 269 bis 273, 1930) gemessen, wobei eine spezielle Meßzelle [Skarp et. al. in Ferroelectric Letters Vol. 06, 000 (1986)] verwendet wird, in der auch die τ- und θ-Werte bestimmt werden. Bei einer Zellenschichtdicke von 2 μm wird durch Scherung eine einheitliche planare Orientierung der Flüssigkristalle in der SmC*-Phase erreicht [SSFLC-Technik, Clark et al., Appl. Phys. Lett. *36*, 899 (1980)]. Nach Anordnung der Meßzelle zwischen zwei gekreuzte Polarisatoren erfolgt die Bestimmung der Schaltzeit mit Hilfe einer Photodiode und die des optischen Neigungswinkels bzw. des Schaltwinkels durch Drehung der Meßzelle von maximalem zu minimalem Lichtdurchgang durch die Meßanordnung. Tabelle II faßt die Ergebnisse für die Mischungen zusammen. Neben den Werten für Ps, τ und 2 θ (jeweils bei 25°C); ist der SmC*-Bereich der jeweiligen Mischung angegeben; die Werte in Klammern geben dabei die unterkühlbare untere Temperaturgrenze des SmC*-Bereichs an.

Tabelle II

| Verbindung Bsp.-Nr. | Anteil der Verb. in mol% | $S_C$*-Bereich der Mischung (°C) | $P_s$ | τ | 2 θ |
|---|---|---|---|---|---|
| 1 | 10 | 7 bis 51 (5) | +4,3 | 215 | 48 |
| 1 | 25 | 7 bis 51 (5) | +13,2 | 55 | 48 |
| 2 | 10 | 14 bis 55 (2) | +5,5 | 85 | 46 |
| 3 | 10 | 5 bis 54 (2) | +6,1 | 98 | 50 |
| 4 | 10 | 8 bis 53 (3) | +5,6 | 100 | 58 |
| 5 | 10 | 12 bis 51 (4) | +3,8 | 100 | 42 |
| 6 | 10 | 13 bis 53 (-) | +5,8 | 285 | 52 |
| 7 | 10 | 12 bis 45 (2) | +2,7 | 220 | 38 |
| 8 | 10 | 18 bis 51 (5) | +3,1 | 106 | 39 |

Phasenfolge der Grundkomponente des Gemischs:

K 13,1 $S_c$ 49,3 $S_A$ 58,9 I
(2,5)

**Patentansprüche für die Vertragsstaaten: BE CH DE FR GB IT LI NL SE**

1. Chirale Verbindungen mit einem mesogenen aromatischen Baustein und einem chiralen Baustein mit einem heterocyclischen Dreiring, dadurch gekennzeichnet, daß in der allgemeinen Formel (I)

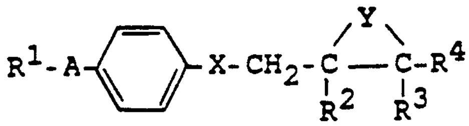
(I)

die Symbole folgende Bedeutung haben:

$R^1$ = geradkettiges oder verzweigtes $(C_1—C_{12})$Alkyl, wobei eine oder zweit nicht-benachbarte $CH_2$-Gruppen durch 0- und/oder S-Atome ersetzt sein können,

A = Diazin-2,5-diyl oder Diazin-3,6-diyl,

X, Y = O und/oder S, und

$R^2$, $R^3$, $R^4$ = unabhängig voneinander H, geradkettiges $(C_1—C_{10})$Alkyl oder verzweigtes $(C_3—C_{10})$Alkyl, wobei $R^2$, $R^3$ und $R^4$ nicht gleichzeitig H sind.

2. Chirale Ether nach Anspruch 1, dadurch gekennzeichnet, daß in der allgemeinen Formel (I) die Symbole folgende Bedeutung haben: $R^1$ = geradkettiges $(C_5$-$C_{11})$Alkyl, wobei eine $CH_2$-Gruppe durch ein O- oder S-Atom ersetzt sein kann, X, Y = O, $R^2$, $R^3$ = H und $R^4$ = geradkettiges oder verzweigtes $(C_3—C_7)$Alkyl.

3. Chirale Ether nach Anspruch 1 oder 2, dadurch gekennzeichnet, daß A = Pyrimidin-2,5-diyl ist.

4. Verdrillbare Flüssigkristall-Phase mit einem Gehalt an mindestens einer chiralen Verbindung, dadurch gekennzeichnet, daß sie mindestens eine chirale Verbindung der allgemeinen Formel (I) nach Anspruch 1 enthält.

5. Verdrillbare Flüssigkristall-Phase nach Anspruch 4, dadurch gekennzeichnet, daß sie neben mindestens einer chiralen Verbindung der allgemeinen Formel (I) nach Anspruch 1 eine Phenylpyrimidin-Verbindung mit $S_c$-Phase enthält.

6. Flüssigkristall-Anzeigeelement enthaltend eine Flüssigkristall-Phase nach Anspruch 4.

7. Verwendung einer chiralen Verbindung gemäß der allgemeinen Formel (I) nach Anspruch 1 zur Umwandlung einer geneigtsmektischen in eine ferroelektrische Flüssigkristall-Phase.

8. Verfahren zur Umwandlung einer geneigt-smektischen in eine ferroelektrische Flüssigkristall-Phase durch Zusatz von mindestens einer chiralen Verbindung, dadurch gekennzeichnet, daß man der Flüssigkristall-Phase mindestens eine Verbindung der allgemeinen Formel (I) nach Anspruch 1 zusetzt.

**Patentansprüche für den Vertragsstaat: AT**

1. Verfahren zum Herstellen von chiralen Verbindungen mit einem mesogenen aromatischen Baustein und einem chiralen Baustein, der einen heterocyclischen Dreiring enthält, dadurch gekennzeichnet, daß man ein mesogenes Phenol oder Thiophenol der Formel II, III oder IV

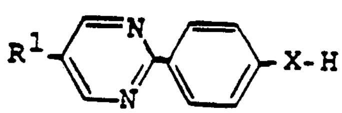
(II)

oder

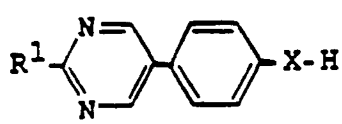
(III)

oder

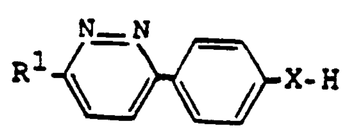
(IV)

mit $R^1$ = geradkettiges oder verzweigtes Alkyl mit 1—12 C-Atomen, wobei eine oder zwei nicht benachbarte $CH_2$-Gruppen durch O und/oder S ersetzt sein können, und

X = O oder S

mit einem chiralen Oxiran oder Thiiran der Formel V

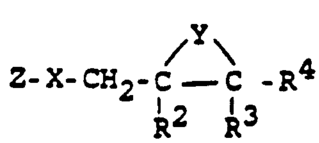
(V)

mit Z = H oder eine nucleofuge Gruppe,
X = O oder S,
Y = O oder S,
$R^2$, $R^3$, $R^4$ = unabhängig voneinander H oder geradkettiges Alkyl mit 1—10 C-Atomen oder verzweigtes Alkyl mit 3—10 C-Atomen, jedoch $R^2$, $R^3$ und $R^4$ nicht gleichzeitig H ist, umsetzt zu Verbindungen der Formel I

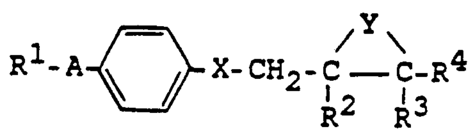

(I)

in der $R^1$, $R^2$, $R^3$, $R^4$, X und Y die oben angegebene Bedeutung haben, und A ein Diazin-2,5-diyl- oder ein Diazin-3,6-diylrest ist.

2. Verfahren nach Anspruch 1, dadurch gekennzeichnet, daß man als mesogenes Phenol ein 2-(4-(Hydroxyphenyl)-5-alkylpyrimidin oder ein 5-(4-(Hydroxyphenyl)-2-alkylpyrimidin einsetzt.

3. Verdrillbare Flüssigkristall-Phase mit einem Gehalt an mindestens einer chiralen Verbindung, dadurch gekennzeichnet, daß sie mindestens eine chirale Verbindung der allgemeinen Formel (I) nach Anspruch 1 enthält.

4. Verdrillbare Flüssigkristall-Phase nach Anspruch 3, dadurch gekennzeichnete, daß sie neben mindestens einer chiralen Verbindung der allgemeinen Formel (I) nach Anspruch 1 eine Phenylpyrimidin-Verbindung mit $S_c$-Phase enthält.

5. Flüssigkristall-Anzeigeelement enthaltend eine Flüssigkristall-Phase nach Anspruch 3.

6. Verwendung einer chiralen Verbindung gemäß der allgemeinen Formel (I) nach Anspruch 1 zur Umwandlung einer geneigt-smektischen in eine ferroelektrische Flüssigkristall-Phase.

7. Verfahren zur Umwandlung einer geneigt-smektischen in eine ferroelektrische Flüssigkristall-Phase durch Zusatz von mindestens einer chiralen Verbindung, dadurch gekennzeichnet, dß man der Flüssigkristall-Phase mindestens eine Verbindung der allgemeinen Formel (I) nach Anspruch 1 zusetzt.

**Patentansprüche für den Vertragsstaat: ES**

1. Verfahren zum Herstellen von chiralen Verbindungen mit einem mesogenen aromatischen Baustein und einem chiralen Baustein, der einen heterocyclischen Dreiring enthält, dadurch gekennzeichnet, daß man ein mesogenes Phenol oder Thiophenol der Formel II, III oder IV

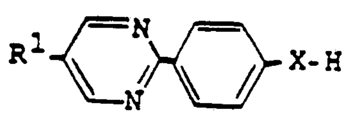

(II)

oder

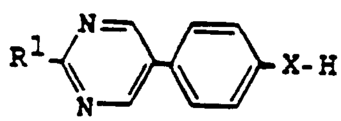

(III)

oder

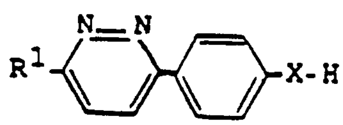

(IV)

mit $R^1$ = geradkettiges oder verzweigtes Alkyl mit 1—12 C-Atomen, wobei eine oder zwei nicht benachbarte $CH_2$-Gruppen durch O und/oder S ersetzt sein können, und
X = O oder S
mit einem chiralen Oxiran oder Thiiran der Formel V

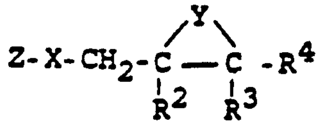

(V)

mit Z = H oder eine nucleofuge Gruppe,
X = O oder S,
Y = O oder S,
$R^2$, $R^3$, $R^4$ = unabhängig voneinander H oder geradkettiges Alkyl mit 1—10 C-Atomen oder verzweigtes

Alkyl mit 3—10 C-Atomen, jedoch $R^2$, $R^3$ und $R^4$ nicht gleichzeitig H ist, umsetzt zu Verbindungen der Formel I

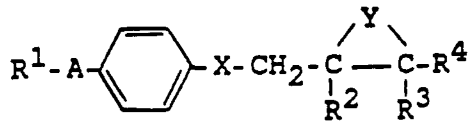

(I)

in der $R^1$, $R^2$, $R^3$, $R^4$, X und Y die oben angegebene Bedeutung haben, und A ein Diazin-2,5-diyl- oder ein Diazin-3,6-diylrest ist.

2. Verfahren nach Anspruch 1, dadurch gekennzeichnet, daß man als mesogenes Phenol ein 2-(4-(Hydroxyphenyl)-5-alkylpyrimidin oder ein 5-(4-(Hydroxyphenyl)-2-alkylpyrimidin einsetzt.

3. Verfahren zum Herstellen einer verdrillbaren Flüssigkristallphase, dadurch gekennzeichnet, daß man eine Mischung aus mindestens einer Verbindung der Formel (I) nach Anspruch 1 und mindestens einer Verbindung mit $S_c$-Phase herstellt.

4. Verfahren zum Herstellen einer verdrillbaren Flüssigkristallphase nach Anspruch 3, dadurch gekennzeichnet, daß man eine Mischung herstellt, die mindestens eine Verbindung der Formel (I) nach Anspruch 1 und mindestens eine Phenylpyrimidinverbindung mit $S_c$-Phase enthält.

5. Verfahren zur Umwandeln einer geneigt-smektischen in eine ferroelektrische Flüssigkristallphase, dadurch gekennzeichnet, daß man eine Verbindung oder eine Mischung, die eine geneigt-smektische Phase bildet, mit mindestens einer Verbindung der Formel (I) nach Anspruch 1 mischt.

**Revendications pour les Etats contractants: BE CH DE FR GB IT LI NL SE**

1. Composés chiraux comportant un composant aromatique mésogène et un composant chiral avec un hétérocycle à trois chaînons, caractérisés en ce que, dans la formule générale (I)

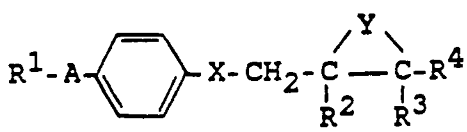

(I)

les symboles ont les significations suivantes:

$R^1$ est un radical alkyle en $C_1$—$C_{12}$ à chaîne droite ou ramifiée, un ou deux groupes $CH_2$ voisins pouvant être remplacés par des atomes d'O et/ou de S,

A est le radical diazinediyle-2,5 ou diazinediyle-3,6,

X, Y est O et/ou S, et

$R^2$, $R^3$, $R^4$, indépendamment les uns des autres, représentent chacun H, un radical alkyle en $C_1$—$C_{10}$ à chaîne droite ou alkyle en $C_3$—$C_{10}$ à chaîne ramifiée, les radicaux $R^2$, $R^3$ et $R^4$ n'étant pas tous simultanément l'hdyrogène.

2. Ethers chiraux selon la revendication 1, caractérisés en ce que, la formule générale (I), les symboles ont les significations suivantes: $R^1$ est un radical alkyle en $C_5$—$C_{11}$ à chaîne droite, un groupe $CH_2$ pouvant être remplacé par un atome O ou S, X, Y = O, $R^2$, $R^3$ = H et $R^4$ est un radical alkyle en $C_3$—$C_7$ à chaîne droite ou ramifiée.

3. Ethers chiraux selon les revendications 1 ou 2, caractérisés en ce que A est le radical pyrimidinediyle-2,5.

4. Phase mésomorphe torsadable, contenant au moins un composé chiral, caractérisée en ce qu'elle contient au moins un composé chiral de formule générale (I) selon la revendication 1.

5. Phase mésomorphe torsadable selon la revendication 4, caractérisée en ce qu'elle contient, outre au moins un composé chiral de formule générale (I) selon la revendication 1, dérivé de phénylpyrimidine ayant une phase $S_c$.

6. Elémente d'affichage à cristaux liquides contenant une phase mésomorphe selon la revendication 4.

7. Utilisation d'un composé chiral de formule générale (I) selon la revendication 1 pour convertir une phase mésomorphe inclinée-smectique en une phase mésomorphe ferroélectrique.

8. Procédé de conversion d'une phase mésomorphe inclinée-smectique en une phase mésomorphe ferroélectrique par addition d'au moins un composé chiral, caractérisé en ce qu'on ajoute à la phase mésomorphe au moins un composé de formule générale (I) selon la revendication 1.

**Revendications pour l'Etat contractant: AT**

1. Procédé de préparation de composés chiraux comportant un composant aromatique mésogène et un composant chiral, lequel contient un hétérocycle à trois chaînons, caractérisé en ce qu'on fait réagir un phénol ou un thiophénol mésogène de formule (II), (III) ou (IV)

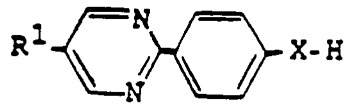

(II)

ou

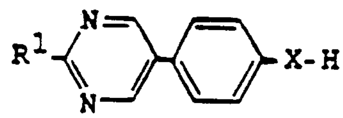

(III)

ou

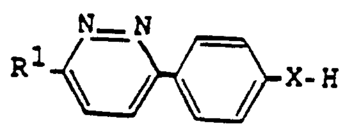

(IV)

où $R^1$ est un radical alkyle à chaîne droite ou ramifiée ayant 1—12 atomes de carbone, un ou deux groupes $CH_2$ non voisins pouvant être remplacés par O et/ou S et

X = O ou S

avec un oxiranne ou un thiiranne chiral de formule (V)

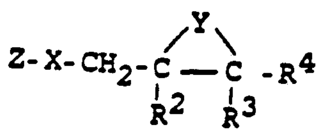

(V)

avec Z = H ou un groupe nucléofuge,

X = O ou S,

Y = O ou S,

$R^2$, $R^3$ et $R^4$, indépendamment les uns des autres, représentent chacun H ou un radical alkyle à chaîne droite ayant de 1—10 atomes de carbone ou un radical alkyle à chaîne ramifiée ayant de 3—10 atomes de carbone, mais $R^2$, $R^3$ et $R^4$ n'étant pas simultanément H,

pour obtenir des composés de formule (I)

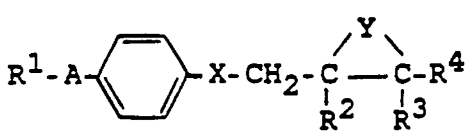

(I)

dans laquelle $R^1$, $R^2$, $R^3$, $R^4$, X et Y ont les significations données ci-dessus, et A et un radical diazinediyle-2,5 ou diazinediyle-3,6.

2. Procédé selon la revendication 1, caractérisé en ce qu'on utilise comme phénol mésogène une (hydroxy-4 phényl)-2 alkyl-5 pyrimidine ou une (hydroxy-4 phényl)-5 alkyl-2 pyrimidine.

3. Phase mésomorphe torsadable, contenant au moins un composé chiral, caractérisée en ce qu'elle contient au moins un composé chiral de formule générale (I) selon la revendication 1.

4. Phase mésomorphe torsadable selon la revendication 3, caractérisée en ce qu'elle contient, outre au moins un composé chiral de formule générale (I) selon la revendication 1, dérivé de phénylpyrimidine ayant une phase $S_c$.

5. Elémente d'affichage à cristaux liquides contenant une phase mésomorphe selon la revendication 3.

6. Utilisation d'un composé chiral de formule générale (I) selon la revendication 1 pour convertir une phase mésomorphe inclinée-smectique en une phase mésomorphe ferroélectrique.

7. Procédé de conversion d'une phase mésomorphe inclinée-smectique en une phase mésomorphe ferroélectrique par addition d'au moins un composé chiral, caractérisé en ce qu'on ajoute à la phase mésomorphe au moins un composé de formule générale (I) selon la revendication 1.

**Revendications pour l'Etat contractant: ES**

1. Procédé de préparation de composés chiraux comportant un composant aromatique mésogène et un composant chiral, lequel contient un hétérocycle à trois chaînons, caractérisé en ce qu'on fait réagir un

phénol ou un thiophénol mésogène de formule (II), (III) ou (IV)

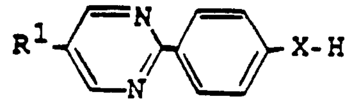

ou

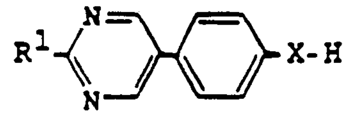

(III)

ou

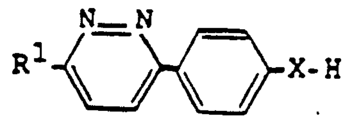

(IV)

où $R^1$ est un radical alkyle à chaîne droite ou ramifiée ayant 1—12 atomes de carbone, un ou deux groupes $CH_2$ non voisins pouvant être remplacés par O et/ou S et

X = O ou S

avec un oxiranne ou un thiiranne chiral de formule (V)

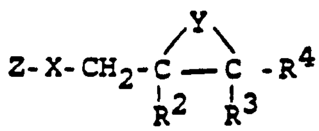

(V)

avec Z = H ou un groupe nucléofuge,

X = O ou S,

Y = O ou S,

$R^2$, $R^3$ et $R^4$, indépendamment les uns des autres, représentent chacun H ou un radical alkyle à chaîne droite ayant de 1—10 atomes de carbone ou un radical alkyle à chaîne ramifiée ayant de 3—10 atomes de carbone, mais $R^2$, $R^3$ et $R^4$ n'étant pas simultanément H,

pour obtenir des composés de formule (I)

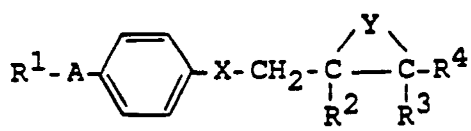

(I)

dans laquelle $R^1$, $R^2$, $R^3$, $R^4$, X et Y ont les significations données ci-dessus, et A est un radical diazinediyle-2,5 ou diazinediyle-3,6.

2. Procédé selon la revendication 1, caractérisé en ce qu'on utilise comme phénol mésogène une (hydroxy-4 phényl)-2 alkyl-5 pyrimidine ou une (hydroxy-4 phényl)-5 alkyl-2 pyrimidine.

3. Procédé de préparation d'une phase mésomorphe torsadable, caractérisé en ce qu'on prépare un mélange d'au moins un composé de formule (I) selon la revendication 1 et d'au moins un composé comportant une phase $S_c$.

4. Procédé de préparation d'une phase mésomorphe torsadable selon la revendication 3, caractérisé en ce qu'on prépare un mélange contenant au moins un composé de formule (I) selon la revendication 1 et au moins un composé phénylpyrimidine comportant une phase $S_c$.

5. Procédé de conversion d'une phase mésomorphe inclinée-smectique en une phase mésomorphe ferroélectrique, caractérise en ce qu'on mélange un composé ou un mélange formant une phase inclinée-smectique à au moins un composé de formule (I) selon la revendication 1.

**Claims for the Contracting States: BE CH DE FR GB IT LI NL SE**

1. A chiral compound having a mesogenic aromatic component and a chiral component having a three-membered heterocyclic ring, wherein, in the formula (I)

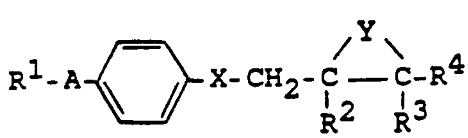

(I)

the symbols have the following meaning:

$R^1$ = straight-chain or branched ($C_1$—$C_{12}$)alkyl, where one or two non-neighboring $CH_2$ groups may be replaced by O and/or S atoms,

A = diazine-2,5-diyl or diazine-3,6-diyl,

X and Y = O and/or S, and

$R^2$, $R^3$ and $R^4$, independently of one another = H, straight-chain ($C_1$—$C_{10}$)alkyl or branched ($C_3$—$C_{10}$)alkyl, where $R^2$, $R^3$ and $R^4$ are not simultaneously H.

2. A chiral ether as claimed in claim 1, wherein, in the formula (I), the symbols have the following meaning: $R^1$ = straight-chain ($C_5$—$C_{11}$)alkyl, where a $CH_2$ group may be replaced by an O or S atom, X and Y = O, $R^2$ and $R^3$ = H and $R^4$ = straight-chain or branched ($C_3$—$C_7$)alkyl.

3. A chiral ether as claimed in claim 1 or 2, wherein A is pyrimidine-2,5-diyl.

4. A twistable liquid-crystal phase containing at least one chiral compound, which phase contains at least one chiral compound of the formula (I) as claimed in claim 1.

5. A twistable liquid-crystal phase as claimed in claim 4, which contains, besides at least one chiral compound of the formula (I) as claimed in claim 1, a phenyl pyrimidine compound having a $S_c$ phase.

6. A liquid-crystal display element containing a liquid-crystal phase as claimed in claim 4.

7. The use of a chiral compound according to the formula (I) as claimed in claim 1 for conversion of a tilted smectic phase into a ferroelectric liquid-crystal phase.

8. A process for converting a tilted smectic phase into a ferroelectric liquid-crystal phase by adding at least one chiral compound, which comprises adding at least one compound of the formula (I) as claimed in claim 1 to the liquid-crystal phase.

**Claims for the Contracting State: AT**

1. A process for preparing chiral compounds having a mesogenic aromatic component and a chiral component which contains a three-membered heterocyclic ring, which comprises reacting a mesogenic phenol or thiophenol of the formula II, III or IV.

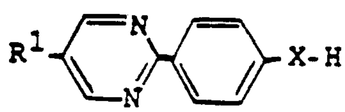

(II)

or

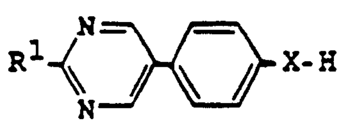

(III)

or

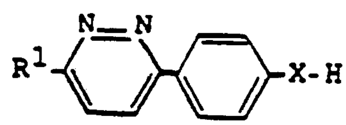

(IV)

in which

$R^1$ = straight-chain or branched alkyl having 1—12 carbon atoms, where one or two non-neighboring $CH_2$ groups may be replaced by O and/or S, and X = O or S

with a chiral oxirane or thiirane of the formula V

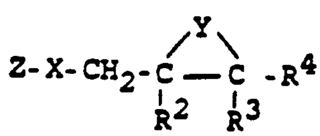

(V)

in which

Z = H or a nucleofugic group,

X = O or S,

Y = O or S, and

$R^2$, $R^3$ and $R^4$, independently of one another, = H or straight-chain alkyl having 1—10 carbon atoms or branched alkyl having 3—10 carbon atoms, but $R^2$, $R^3$ and $R^4$ are not simultaneously H,

to give compounds of the formula I

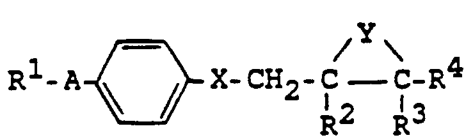

(I)

in which $R^1$, $R^2$, $R^3$, $R^4$, X and Y have the abovementioned meanings, and A is a diazine-2,5-diyl or a diazine-3,6-diyl radical.

2. The process as claimed in claim 1, wherein the mesogenic phenol employed is a 2-(4-(hydroxyphenyl)-5-alkylpyrimidine or 5-(4-(hydroxyphenyl)-2-alkylpyrimidine.

3. A twistable liquid-crystal phase containing at least one chiral compound, which phase contains at least one chiral compound of the formula (I) as claimed in claim 1.

4. A twistable liquid-crystal phase as claimed in claim 4, which contains, besides at least one chiral compound of the formula (I) as claimed in claim 1, a phenyl pyrimidine compound having a $S_c$ phase.

5. A liquid-crystal display element containing a liquid-crystal phase as claimed in claim 3.

6. The use of a chiral compound according to the formula (I) as claimed in claim 1 for conversion of a tilted smectic phase into a ferroelectric liquid-crystal phase.

7. A process for converting a tilted smectic phase into a ferroelectric liquid-crystal phase by adding at least one chiral compound, which comprises adding at least one compound of the formula (I) as claimed in claim 1 to the liquid-crystal phase.

**Claims for the Contracting State: ES**

1. A process for preparing chiral compounds having a mesogenic aromatic component and a chiral component which contains a three-membered heterocyclic ring, which comprises reacting a mesogenic phenol or thiophenol of the formula II, III or IV.

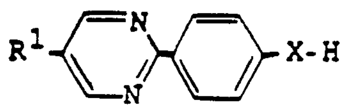

(II)

or

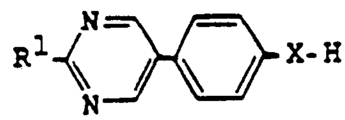

(III)

or

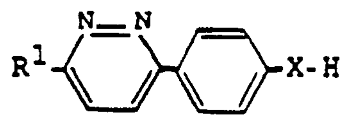

(IV)

in which

$R^1$ = straight-chain or branched alkyl having 1—12 carbon atoms, where one or two non-neighboring $CH_2$ groups may be replaced by O and/or S, and X = O or S

with a chiral oxirane or thiirane of the formula V

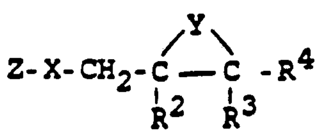

(V)

in which

Z = H or a nucleofugic group,

X = O or S,

Y = O or S, and

$R^2$, $R^3$ and $R^4$, independently of one another, = H or straight-chain alkyl having 1—10 carbon atoms or branched alkyl having 3—10 carbon atoms, but $R^2$, $R^3$ and $R^4$ are not simultaneously H,

to give compounds of the formula I

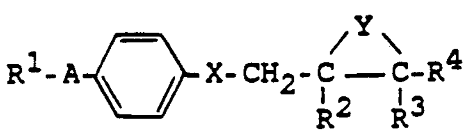

(I)

in which $R^1$, $R^2$, $R^3$, $R^4$, X and Y have the abovementioned meanings, and A is a diazine-2,5-diyl or a diazine-3,6-diyl radical.

2. The process as claimed in claim 1, wherein the mesogenic phenol employed is a 2-(4-(hydroxyphenyl)-5-alkylpyrimidine or 5-(4-(hydroxyphenyl)-2-alkylpyrimidine.

3. A process for preparing a twistable liquid-crystal phase which comprises preparing a mixture from at least one compound of the formula (I) as claimed in claim 1 and at least one compound having a $S_c$ phase.

4. The process for preparing a twistable liquid-crystal phase as claimed in claim 3 wherein a mixture is prepared which contains at least one compound of the formula (I) as claimed in claim 1 and at least one phenyl pyrimidine compound having a $S_c$ phase.

5. A process for converting a tilted smectic phase into a ferroelectric liquid-crystal phase which comprises mixing a compound or a mixture which forms a tilted smectic phase with at least one compound of the formula (I) as claimed in claim 1.